# EUROPEAN PATENT APPLICATION

(11) **EP 2 662 086 A1**
(43) Date of publication of application: **13.11.2013**
(21) Application number: 12167203.4
(22) Date of filing: 08.05.2012
(51) Int. Cl.: A61K 36/45, A61K 36/28, A61P 31/04

(54) **Composition for the treatment or prevention of urinary tract infections and dosage form**

(71) Applicant: Progressare Medinvest B.V., 1019 HC Amsterdam (NL)
(72) Inventor: Hendriks, Maikel, 1019 HC Amsterdam (NL); Bouter, Pieternella, 1019 HC Amsterdam (NL); Van den Ende, Maarten, 1019 HC Amsterdam (NL)
(74) Representative: Jansen, Bart Antonius Johannes

(57) **Abstract**

The invention relates to a composition for the treatment or prevention of urinary tract infections, and a dosage for comprising such a composition. The composition comprises an anti-adhesive comprising an herbal extract or mixture of extracts having an anti-adhesive effect bacteria in the urinary tract, and at least one diuretic. Of particular interest are combinations of *Vaccinium macrocarpon* (cranberry) and *Solidago virgaurea* (goldenrod), and *Vaccinium macrocarpon* combined with *Juniperus* (juniper).

## Description

### FIELD OF THE INVENTION

The invention relates to a composition for the treatment or prevention of urinary tract infections, and a dosage for comprising such a composition.

### BACKGROUND OF THE INVENTION

Cranberry juice is believed to have advantageous properties with respect to bacterial infections in the urinary system. It has been postulated that its effect can be ascribed to proanthocyanidins and other assumed active ingredients that may inhibit the adhesion of E coli bacteria to the bladder.

The properties ascribed to cranberry juice have led to several concentrated cranberry extracts commercially available as food supplements, in particular in the form of tablets and gelatin capsules. The effectiveness of commercially available extracts may vary, depending on the extraction method, and the quality and type of cranberry used.

Several different types of cranberries are known, in particular Vaccinium oxycoccos or Oxycoccus palustris (Common Cranberry or Northern Cranberry, Vaccinium microcarpum or Oxycoccus microcarpus (Small Cranberry). Vaccinium macrocarpon or Oxycoccus macrocarpus (Large cranberry, American Cranberry, Bearberry) and Vaccinium erythrocarpum or Oxycoccus erythrocarpus (Southern Mountain Cranberry).

### OBJECT AND SUMMARY OF THE INVENTION

It is an object of the invention to enable an improved treatment or prevention of urinary tract infections.

The invention provides a composition for the treatment or prevention of urinary tract infections (UTI), comprising an herbal extract or mixture of extracts having anti-adhesive effect bacteria in the urinary tract, in particular E. coli, at least one diuretic, and optionally other active ingredients and/or excipients.

The combination of an herbal extract preventing or reversing the adhesion of bacteria with a diuretic showed a synergistic effect in treating and/or preventing urine tract infections. An improvement in beneficial effect was noted for a combination of cranberry extract and a diuretic was larger than treatment compared to the cranberry extract as such.

Several herbal extracts having an anti-adhesive effect bacteria in the urinary tract can be used, preferably the various types of cranberry and blueberry.

Other ingredients and excipients may include for instance fillers to enable the easy processing into tablets, such as magnesium stearate, sucrose, lactose; starches, cellulose or modified cellulose such as microcrystalline cellulose and cellulose ethers such as hydroxypropyl cellulose (HPC) and hydroxypropyl methylcellulose (HPMC); and sugar alcohols such as xylitol, sorbitol and maltitol. Flavors to improve palatability may be used, including various fruit flavorings such as strawberry, peach, raspberry or apricot. Colorants may also be added for a distinguishing visual appearance.

It is preferred if the herbal extract comprises at least on extract selected from the group consisting of *Vaccinium macrocarpon* (cranberry), Vaccinium oxycoccos or Oxycoccus palustris (Common Cranberry or Northern Cranberry, Vaccinium microcarpum or Oxycoccus microcarpus (Small Cranberry). Vaccinium macrocarpon or Oxycoccus macrocarpus (Large cranberry, American Cranberry, Bearberry), Vaccinium erythrocarpum or Oxycoccus erythrocarpus (Southern Mountain Cranberry) and Vaccinium angustifolium (Blueberry).

Most preferably, the herbal extract comprises at least an extract of *Vaccinium macrocarpon* (cranberry). This cranberry has a good availability and showed excellent properties against urinary tract infections.

The diuretic may be either natural of synthetic. Well known diuretics include hydrochlorothiazide, acetazolamide. methazolamide, spironolactone, amiloride, triamterene. Natural diuretics or mixtures thereof are preferred, though.

It is advantageous if the diuretic comprises at least one extract derived from the group consisting of *Solidago virgaurea* (goldenrod), *Orthosiphon stamineus* (java tea), *Levisticum officinale* (lovage), *Petroselinum crispum* (parsley), *Asparagus officinalis* (asparagus), and *Juniperus communis* (Common Juniper). Extracts of these herbs are commercially available.

Most preferably, the diuretic comprises an extract derived from *Solidago virgaurea* (goldenrod) or *Juniperus* (juniper). (Goldenrod is also known as Golden Rod, Verge d'Or, Solidago, Goldruthe, Woundwort, and Aaron's Rod. Various types of juniper may be used, in particular *Juniperus communis, Juniperus chinensis or Juniperus angosturana.*

Preferably, the composition comprises a combination of extracts of *Vaccinium macrocarpon* (cranberry) and *Solidago virgaurea* (goldenrod).

Another preferred embodiment combines of *Vaccinium macrocarpon* (cranberry) and *Juniperus* (juniper). Juniper showed to particularly advantageous when combined with cranberry. A disadvantage is that Juniperus is believed to cause health risks for pregnant women for babies during the lactation period.

It is preferred if the amount of diuretic by weight is lower than the amount of anti-adhesive. Depending on the type of diuretic used, the amount of diuretic by weight could be at least 5 times lower than the amount of anti-adhesive. This ensures the anti-adhesive effect prevails over the diuretic effect. Having more diuretic than anti-adhesive increases the risk of a prevailing diuretic effect, which may be inconvenient for the user.

It is advantageous if the composition is an essentially solid composition. Solid compositions have an improved storage life over liquid compositions. Preferably, the composition is a lyophilized powder. Lyophilized or freeze-dried compositions are relatively easy to dose and may be used for the preparation of dosage forms such as capsules and tablets.

The invention further comprises a capsule or tablet comprising a composition according to any of the preceding claims. The dosage form, preferably a capsule or a tablet, comprising a composition as described above, preferably comprises a dose of at least 400 mg of extract of *Vaccinium macrocarpon* (cranberry), and at least 10 mg of *Solidago virgaurea,* and optionally other active ingredients and/or excipients.

It is preferred if the capsule is essentially free of gelatin. Gelatin and other animal-derived materials have an increased risk of contamination and therefore require additional safety checks.

Preferably, the dosage form is a capsule made of a water-soluble polymer material. It is advantageous if the water-soluble polymer material comprises at least one compound selected from the group consisting of methyl cellulose, hydroxyethyl cellulose, hydroxypropyl cellulose (HPC), hydroxyethyl methyl cellulose, hydroxypropyl methylcellulose (HPMC) and carboxymethyl cellulose (CMC).

### DESCRIPTION OF PREFERRED EMBODIMENTS

The invention will now be further elucidated by the following non-limiting examples. All ingredients are commercially available.

### Formulations

### Example 1

This example uses a combination of cranberry extract (Vaccinium macrocarpon) and golden rod (Solidago virgaurea). Purified potato starch (Amylum solani) was used as a filler. The ingredients were mixed using conventional techniques, and encapsulated in hydroxypropyl methylcellulose (HPMC)-based two-piece capsules. The table indicates the components of the composition in a single dose hydroxypropyl methylcellulose capsule.

| | **%w/w** | **mg** |
|---|---|---|
| Cranberry extract | 70 | 500 |
| Solidago virgaurea extract | 10 | 75 |
| Amylum solani | 4 | 25 |

### Example 2

| | **%w/w** | **mg** |
|---|---|---|
| Cranberry extract | 70 | 500 |
| Solidago virgaurea extract | 7 | 50 |
| Solidago virgaurea herb | 7 | 50 |

### Example 3

The examples 3 is a powder formulation that usse a combination of cranberry extract *(Vaccinium macrocarpon)* and juniper *(Juniperus communis).* Purified potato starch (*Amylum solani*) was used as a filler. The ingredients were mixed using conventional techniques, and encapsulated in hydroxypropyl methylcellulose (HPMC)-based two-piece capsules. The table indicates the components of the composition and the hydroxypropyl methylcellulose capsule.

Juniperus extracts are commercially available in 1:5 to 1:25 extracts. The extract used herein is a 20:1 extract, being an equivalent of 1 gram of dried juniper fruit per powder dose.

A dosage of at least 2 doses per day (equivalent of 2 grams of dried juniper fruit) is recommended for a good preventive or curative effect when combined with cranberry extract.

| | **%w/w** | **mg** |
|---|---|---|
| Cranberry extract | 70 | 500 |
| Juniperus extract | 10 | 100 |
| Amylum solani | 4 | 25 |

### Ex-vivo E.coli anti-adhesion test

The effect of cranberry extracts was tested ex vivo on E. coli in an ex-vivotests.

A randomized, double-blinded, placebo controlled crossover trial was conducted to determine the ex vivo uropathogenic bacterial P-type anti-adhesion activity (AAA) in human urine following consumption of either 500-mg cranberry extract capsule as shown in example 1, or a placebo capsule with a 7-day wash-out period, measured over a 60-hr time frame with product consumed at the beginning of the test period only.

The cranberry capsules had a higher total AAA than Placebo over all time periods for all participants combined. These results were highly significant (p<0.0001). By time period, AAA of cranberry capsules was significantly higher than Placebo from 9 to 36 hours after ingestion (p<0.0001 at 9 and 24 hours, and p=0.0003 at 36 hours). Placebo ingestion resulted in no significant AAA at any time period.

The experiment shows that the product indeeed has an anti-adhesive effect for E.coli in the urinary tract.

### E. coli in vivo

A dose-dependent study was also carried out to understand the impact and safety profile of a standardized whole cranberry product comparable to the formulation in the examples on reducing the recurrences of symptomatic UTI in culture-positive subjects. A 90- day randomized clinical trial including an untreated control group with a total of 60 female subjects between 18-40 years of age was conducted. The safety was assessed by evaluation of biochemical and hematological parameters on days 10, 30, 60 and 90 during the study, comparing the values with those at the baseline. At the end of the study, there was significant reduction (p<0.05) in the subjects positive for E. coli in both the high dose and low dose treatment groups, compared to baseline evaluation. Symptomatic relief was also reported in the low and high dose treatment groups, while none was reported by subjects in the untreated control group. In conclusion, the standardized cranberry product was effective in safely reducing the number of E. coli positive subjects at both the 500 mg and 1000 mg dose levels and in ameliorating the symptoms of UTI in these subjects.

### Efficacy test women

In a randomized double blind trial, the efficacy of cranberry extracts combined with juniper extracts was tested against a placebo as a prophylactic against urinary tract infections. For the test, a daily dose of 1000 mg cranberry extract and 100 mg juniper extract was used, based on the daily intake of two capsules according to example 3.

The efficacy of the formulation was tested in women with at least one UTI in the previous 12 months. The participants were randomized to either treatment with 1000 mg/day cranberry extract and 100 mg juniper extract (n=59) or placebo only containing filler material (n=66). During 6 months, 18 UTI events occurred in the placebo group and 8 UTI events in the treated group. This indicates that the combination of cranberry and juniper is effective in

### Qualitative comparative tests

In an initial comparative test, test persons regularly suffering from bladder infections were given cranberry extract capsules, and indicated that these cranberry extract tablets helped to aleviate bladder infection, and also the bladder infections appeared to be less frequent.

Test persons from the same group were also given the tablets having a combination of cranberry with golden rod or cranberry with juniper. 70% of the test persons indicated that they experienced an improved positive effect on their bladder infections.

## Claims

1. Composition for the treatment or prevention of urinary tract infections, comprising
- an anti-adhesive comprising an herbal extract or mixture of extracts having an anti-adhesive effect bacteria in the urinary tract,
- at least one diuretic, and
- optionally other active ingredients and/or excipients.

2. Composition according to claim 1, wherein the herbal extract comprises at least one extract selected from the group consisting of *Vaccinium macrocarpon* (cranberry), Vaccinium oxycoccos or Oxycoccus palustris (Common Cranberry or Northern Cranberry, Vaccinium microcarpum or Oxycoccus microcarpus (Small Cranberry). Vaccinium macrocarpon or Oxycoccus macrocarpus (Large cranberry, American Cranberry, Bearberry), Vaccinium erythrocarpum or Oxycoccus erythrocarpus (Southern Mountain Cranberry) and Vaccinium angustifolium (Blueberry).

3. Composition according to claim 2, wherein the herbal extract comprises at least an extract of *Vaccinium macrocarpon* (cranberry), Vaccinium oxycoccos or Oxycoccus palustris (Common Cranberry or Northern Cranberry, Vaccinium microcarpum or Oxycoccus microcarpus (Small Cranberry). Vaccinium macrocarpon or Oxycoccus macrocarpus (Large cranberry, American Cranberry, Bearberry), Vaccinium erythrocarpum or Oxycoccus erythrocarpus (Southern Mountain Cranberry) and Vaccinium angustifolium (Blueberry).

4. Composition according to any of the preceding claims, wherein the diuretic is a natural diuretic.

5. Composition according to claim 4, where the diuretic comprises at least one extract derived from the group consisting of *Solidago virgaurea* (goldenrod), *Orthosiphon stamineus* (java tea), *Levisticum officinale* (lovage), *Petroselinum crispum* (parsley), *Asparagus officinalis* (asparagus), and *Juniperus communis* (Common Juniper).

6. Composition according to claim 5, wherein the diuretic comprises an extract derived from *Solidago virgaurea* (goldenrod) or *Juniperus* (juniper).

7. Composition according to any of the preceding claims, wherein the composition comprises a combination of extracts of *Vaccinium macrocarpon* (cranberry) and *Solidago virgaurea* (goldenrod).

8. Composition according to any of the preceding claims, wherein the composition comprises a combination of extracts of *Vaccinium macrocarpon* (cranberry) and *Juniperus* (juniper).

9. Composition according to any of the preceding claims, wherein the amount of diuretic by weight is lower than the amount of anti-adhesive.

10. Composition according to any of the preceding claims, wherein the composition is an essentially solid composition.

11. Composition according to claim 10, wherein the composition is a lyophilized powder.

12. Dosage form, preferably a capsule or a tablet, comprising a composition according to any of the preceding claims.

13. Dosage form according to claim 12, wherein the dosage form comprises a dose of at least 400 mg of extract of *Vaccinium macrocarpon* (cranberry), and at least 10 mg of *Solidago virgaurea,* and optionally other active ingredients and/or excipients.

14. Capsule according to any of the claims 11-13, wherein the dosage form is a capsule made of a water-soluble polymer material.

15. Capsule according to claim 14, wherein the water-soluble polymer material comprises at least one compound selected from the group consisting of methyl cellulose, hydroxyethyl cellulose, hydroxypropyl cellulose (HPC), hydroxyethyl methyl cellulose, hydroxypropyl methylcellulose (HPMC) and carboxymethyl cellulose (CMC).
